# EUROPEAN PATENT APPLICATION

(11) **EP 3 674 978 A1**
(43) Date of publication of application: **01.07.2020**
(21) Application number: 19220119.2
(22) Date of filing: 30.12.2019
(51) Int. Cl.: G06K 9/00

(54) **SYSTEM AND METHOD FOR DETECTING REACTION TIME OF A DRIVER**

(30) Priority: 31.12.2018 IN 201811050067; 31.12.2018 IN 201813050098
(71) Applicant: The Hi-Tech Robotic Systemz Ltd, 122001 Gurugram (IN)
(72) Inventor: KAPURIA, Anuj, 122001 Gurugram (IN); VIJAY, Ritukar, 122001 Gurugram (IN)
(74) Representative: Dantz, Dirk

(57) **Abstract**

The present invention provides a method and system for monitoring driver inattentiveness using plurality of physiological factors of the driver. In this method, captured images and/or short videos are used to determine the physiological factors of the driver. The first physiological factor from the plurality of physiological factors is used to determine the level of drowsiness and/or inattentiveness of the driver, which further supported by the second physiological factor. The data generated from the analysis of first and second physiological factors is further utilized to generate a predictive warning to the driver. The intensity level of the warning is varied based on the analyzed level of inattentiveness of the driver. The warning may be an audio warning, a visual warning, an audio-visual warning, haptic warning like vibration, etc.

## Description

### FIELD OF INVENTION

The present invention relates to autonomous driving vehicles and more particularly related to monitoring drivers during vehicle driving and capturing various physiological factors to identify driver state or predict driver state.

### BACKGROUND OF THE INVENTION

Modern vehicles are generally equipped with various types of monitoring systems, such as cameras, or video recorders to monitor surrounding environment of vehicles and provide a driver of a vehicle with useful data regarding the surrounding environment for improved driving. Such monitoring systems may be installed, for instance, on a roof of the vehicle or on the front portion, back portion of the vehicle to have a broad view of the surrounding environment and capture data associated with objects, pedestrians or vehicles within the surrounding environment.

In addition, the monitoring systems may also monitor the driver of the vehicle for facial pose and gaze. For instance, the driver may be monitored for orientation of the face and the gaze to be in a forward direction and determine if the driver is paying attention on the road. The collected data is then subjected to processing to derive meaningful information that may be used in assisting the driver for navigation, changing lanes, and averting a potential collision. An event, such as an approaching vehicle, a pedestrian on the road may be detected and a warning may be issued to the driver to help the driver initiate a precautionary action.

However, such monitoring systems, on many occasions, fail to detect events with accuracy due to various factors such as incomplete data or incorrect data, and issue false or irrelevant warnings to the driver. These warnings are generally issued at high volumes to alert the driver that on many instances may startle or distract the driver, thereby inciting a sudden action that could be potentially harmful for the safety of the driver. Further, such irrelevant warnings issued regularly at high volumes may cause a general discomfort, and impact driving of the driver. Therefore, the monitoring systems are not efficient in detecting events and issuing warning to the drivers for enhancing driving experience and safety.
Therefore, there is a need of an efficient system for maintaining driver attentiveness even while the driver is not participating in the controlling of the vehicle.

### SUMMARY OF THE INVENTION

This summary is provided to introduce concepts related to monitoring driver inattentiveness using physiological factors. This summary is not intended to identify essential features of the claimed subject matter nor is it intended for use in determining or limiting the scope of the claimed subject matter.

In an example implementation of the present subject matter, a method for monitoring the inattentiveness of a vehicle driver is provided. The method includes steps of capturing and storing the images of the driver. Further, the method includes simultaneous analysis of the stored images of the driver is used in the next step of the method to generate a predictive warning of the inattentiveness of the driver based on the captured images. Thereafter, the analysis of the captured images is used to extract the plurality of physiological factors of the driver. The inattentiveness of the driver is determined based on a first physiological factor from the plurality of physiological factors. Further, the second physiological factor is determined to support the first physiological factor. By way of example, physiological factors such as heart rate variability and pupillary light reflex are potentially interrelated, and it can be simultaneously measured for physiological analysis of the driver.

Although the present subject matter has been described with reference to an integrated system comprising the modules, the present subject matter may also be applicable to provide a warning to an inattentive driver of the vehicle by the modules placed at different areas within an autonomous vehicle, wherein the modules are communicatively coupled to each other.

In an example implementation of the present subject matter, an ADAS includes a drive mode monitoring module, driver monitoring module, an environment condition module, a vehicle information module, a processor coupled to the different monitoring modules, and a warning generating module coupled to the processor. In accordance with an embodiment of the invention, driver monitoring module captured various images and/or videos to determine the plurality of physiological factors of the driver which further stored in the memory. In the system, the processing unit is analyzed the captured images continuously to determine the plurality of physiological factors of the driver. Further, the processing unit may use any physiological factor as a first physiological factor to determine the inattentiveness of the driver. Furthermore, the second physiological factor is also analyzed to support the first physiological factor to determine the inattentiveness of the driver. The physiological factors enable early prediction of drowsiness and inattentiveness of the drivers.

Thus, the present subject matter provides efficient techniques for detecting the inattentiveness of a driver using different physiological factors. The techniques provide an adaptive warning to the driver of the vehicle, wherein the intensity level of the warning is varied based on the analyzed level of inattentiveness.

In an embodiment of the invention, the driving behavior is determined from data received from the driver monitoring module, environment condition module, and vehicle information module. Also, the information of historical reactions may also be fetched from memory for reaction time calculation. Determination of reaction time is done based on what state the driver is currently in, what are the physiological readings for the same and how the driver has been reacting to situations in the current journey. The reaction time may be utilized to better equip the system for any situations that may come up in the journey. The processing module may predict reaction time adjustments from historical data stored that it may fetch from a remote server connected to it. Early predictive reaction time adjustments may be provided to the driver based on his current physiological and driving behavior.

Other and further aspects and features of the disclosure will be evident from reading the following detailed description of the embodiments, which are intended to illustrate, not limit, the present disclosure.

### BRIEF DESCRIPTION OF ACCOMPANYING DRAWINGS

The foregoing summary, as well as the following detailed description of various embodiments, is better understood when read in conjunction with the drawings provided herein. For the purpose of illustration, there is shown in the drawing's exemplary embodiments; however, the presently disclosed subject matter is not limited to the specific methods and instrumentalities disclosed.
FIG.1 is a block diagram of an autonomous vehicle and its subsystems, in accordance with an embodiment of the invention;
FIG. 2A is a line diagram of a vehicle dashboard, in accordance with an embodiment of the invention;
FIG. 2B is a line diagram of a driver monitoring module, in accordance with an embodiment of the invention;
FIG. 3 is a block diagram of a system for monitoring driver during a drive session and generate a predictive warning of inattentiveness, in accordance with an embodiment of the invention;
FIG. 4 is flow chart depicting an overall method of providing early warning, in accordance with an embodiment of the invention;
FIG. 5 is a flow chart illustrating a method of providing early warning, in accordance with an embodiment of the invention.
FIG. 6 is flow chart depicting an overall method of determining and adjusting reaction time of a driver, in accordance with an embodiment of the invention;

### DETAILED DESCRIPTION OF INVENTION

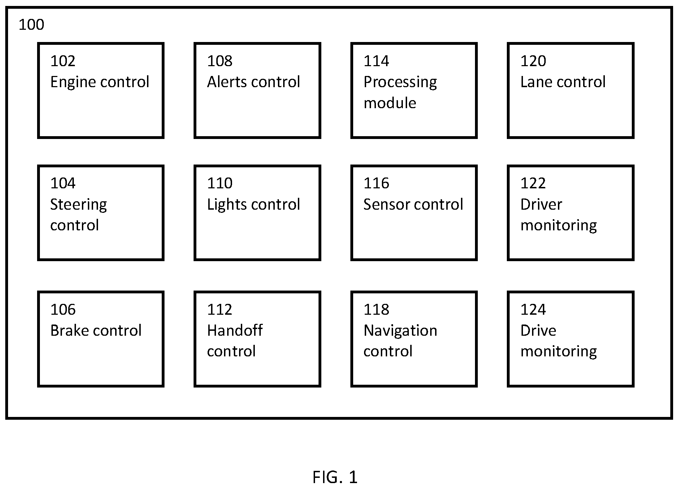

FIG. 1 shows a block diagram of an autonomous vehicle 100 (termed as vehicle 100 interchangeably within the description) and its various subsystems, in accordance with an embodiment of the invention. According to an embodiment of the invention, the autonomous vehicle 100 may be a fully or a semi-autonomous vehicle. The autonomous vehicle 100 includes multiple sub systems to control various important processes and functions. The autonomous vehicle 100 may include Engine control module 102, Steering control module 104, Brake control module 106, Alerts control module 108, Lights control module110, Handoff control module 112, Processing module 114, Sensor control module 116, Navigation control module 118, Lane control module 120, Driver monitoring module 122, and Drive monitoring module 124.

Engine control module 102 controls various functions and processes of an engine of the vehicle 100. Functions and processes to be controlled may be speed of rotation, engine condition, servicing requirements, load on engine, power of engine, etc.

Steering control module 104 may help in movement of the vehicle 100. The steering control module 104 helps vehicle 100 to be driven and controlled in transverse and longitudinal direction. Steering module 104 may include actuators that may control the steering module 104 in autonomous mode.

Brake control module 106 of the autonomous vehicle 100 may help in braking function of the vehicle 100. Brake control module 106 may control brakes of all four wheels using disc or horse-shoe brake parts. The brake control module 106 may also include actuators connected to brake parts in order to control braking while in autonomous drive mode.

Alerts control module 108 may control various alerts to be provided during various situations. The alerts may include ranging from servicing requirement of the vehicle 100 to lane change assist alerts during manual mode.

Lights control module 110 may control various lighting functions of the vehicle 100. The lighting functions may be for example, switching on lights while ambient light is below a threshold or changing low beam to high beam while road is empty and high beam is required due to night lighting conditions on road.

Handoff control module 112 takes care of drive handling control of the vehicle 100. The handoff control module 112 may be responsible for switching control of the vehicle 100 to autonomous from manual or vice versa. The handoff control module 112 takes over full control function of the vehicle 100 while switching to autonomous mode.

Processing module 114 provides computing power to the vehicle 100. The processing module 114 helps the vehicle 100 in all the calculations required for autonomous, or semi-autonomous driving modes as well. It may also be useful in manual driving mode as well wherein the processing module 114 may process route calculations, fuel requirements, etc. In autonomous mode, the processing module 114 may take in data from various sensors and use the sensor data for efficient drive control during autonomous drive mode.

Sensor control module 116 collects data from the physical sensors provided all over the vehicle 100. The sensors may be RADAR sensors, ultrasonic sensors, LiDAR sensor, proximity sensors, weather sensors, heat sensors, tire pressure sensors, etc. the sensor control module 116 in association with the processing module 114 may also calibrate the sensors regularly due to dynamic environment around the vehicle 100.

Navigation control module 118 helps the autonomous vehicle 100 during active autonomous drive mode in navigation. In general, the navigation control module 118 may include route calculation, maps, road sign identification etc. for efficient navigation of the vehicle 100.

Lane control module 120 may help the vehicle 100 to control lane changing and drive within a lane as marked on the road. Lane control module 100 may be take input data from image and RADAR sensors to identify lanes and help the vehicle to change lanes during an active autonomous drive mode.

Driver monitoring module 122 collects data about driver during an active autonomous drive mode, semiautonomous mode and manual mode. It collects data about driver like expressions, eye gaze, emotions, facial identity etc. Data about driver may be collected using various cameras facing into a cabin of the vehicle 100.

Drive monitoring module 124 collects data about drive of the vehicle 100. The drive may be autonomous drive or manual drive. Data collected may be like drive behavior in various situations, various conditions, confidence level, stress induced mistakes etc. Drive monitoring module 124 may help in ascertaining drive behavior during the drive that may be kept for records and utilized for improving future drive interactions, and mistakes while driving the vehicle 100. Furthermore, collected data, such as the deviation of behavioral trends, spoken words, gaze monitoring and / or environmental conditions within the vehicle are used to provide useful data regarding the state of the driver.

It is to be noted, that the vehicle 100 may further include some more modules that may help in functioning of the vehicle 100 and some modules as mentioned above may be combined to perform similar functions.

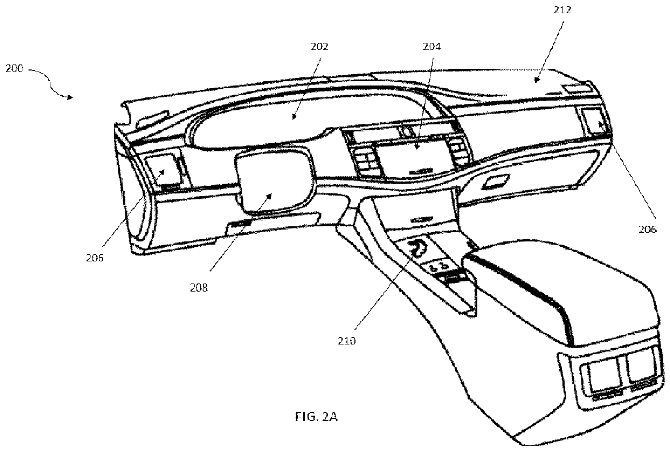

FIG. 2A is a line diagram of a dashboard 200 of a vehicle, in accordance with an embodiment of the invention. The dashboard 200 includes an instrument cluster 202, an infotainment system 204, Air conditioning vents 206, steering space 208, and a central console 210.

The instrument cluster 202 may include indicators (not shown in figure) for speed, distance, rotations per minute, fuel indications, heating indications, etc. The infotainment system 204 provides various entertainment features like music system, navigation, various alerts, etc. to the driver of the vehicle. Air conditioning vents 206 may be provided in order to control climate of a cabin of the vehicle. As depicted there may be multiple air conditioning vents provided within the dashboard 200. The dashboard 200 may also include a steering space 208 wherein steering wheel of the vehicle is accommodated. Further, there may also be provided a central console 210 for driver's use like storage, bottle holders, etc.

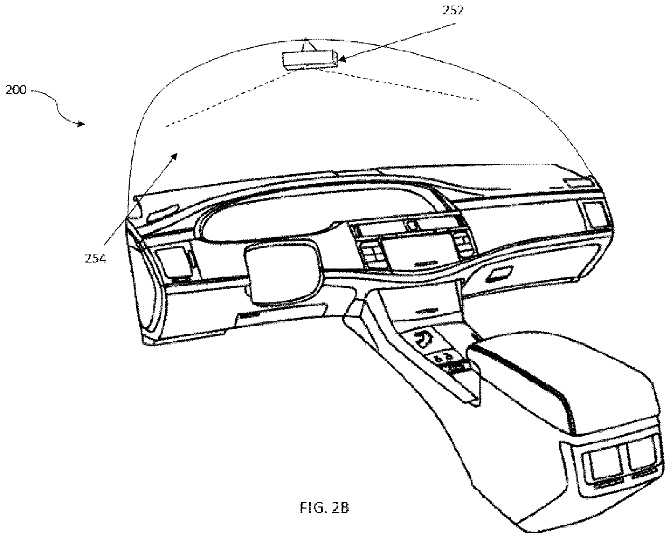

FIG. 2B is a line diagram of the dashboard 200 of the vehicle including a driver monitoring module 252 placed near roof of the vehicle 200 in accordance with an embodiment of the invention. The driver monitoring module 252, may be configured to take images of the driver while driving and during various situations faced during the journey.

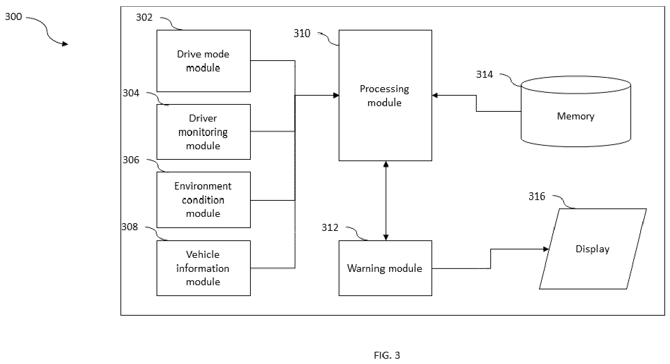

FIG. 3A is a block diagram of a system 300 for monitoring a driver during a drive session and generate a predictive warning of inattentiveness, in accordance with an embodiment of the invention. The system 300 may include multiple modules like a drive mode monitoring module 302, a driver monitoring module 304, an environment condition module 306, a vehicle information module 308, a processing module 310, a warning module 312, a memory 314, and a display 316.

In an implementation, some of the modules such as the drive mode module 302, the driver monitoring module 304, the environment condition module 306, the vehicle information module 308, the processing module 310, the training module 312 may include routines, programs, objects, components, data structure and the like, which perform particular tasks or implement particular abstract data types. The modules may further include modules that supplement applications on the processing module 310, for example, modules of an operating system. Further, the modules can be implemented in hardware, instructions executed by a processing unit, or by a combination thereof.

In another aspect of the present subject matter, the modules may be machine-readable instructions which, when executed by a processor/processing module, perform any of the described functionalities. The machine-readable instructions may be stored on an electronic memory device, hard disk, optical disk or other machine-readable storage medium or non-transitory medium. In an implementation, the machine-readable instructions can also be downloaded to the storage medium via a network connection. Memory 314 may be without limitation, memory drives, removable disc drives, etc., employing connection protocols such as serial advanced technology attachment (SATA), integrated drive electronics (IDE), IEEE-1394, universal serial bus (USB), fiber channel, small computer systems interface (SCSI), etc. The memory drives may further include a drum, magnetic disc drive, magneto-optical drive, optical drive, redundant array of independent discs (RAID), solid-state memory devices, solid-state drives, etc.

Drive mode monitoring module 302 determines, the active driving mode. Driving mode may be manual, semi-autonomous or autonomous. The drive mode module 302 may accept input from user to activate any of the three drive modes. The drive mode module 302 may be a touch button or a physical button or the like. A driver may provide input to the drive mode module 302 to initiation of the driving mode as required by the driver. Driver monitoring module 304 is positioned to face the driver of a vehicle and monitors presence of the driver. The driver monitoring module 304 may be a combination of image sensors, occupancy sensors, thermal sensors etc. In operation, the driver monitoring module 304 may sense presence or absence of the driver. The driver's presence may be determined using techniques like motion detection, occupancy sensing, thermal vision etc. The driver monitoring module 304, extracts attributes of the driver, once it is established that the driver is present, within the vehicle to identify the driver. Extracted attributes may include, but not limited to a facial scan, a retinal scan, thermal signatures, a fingerprint scan etc. In another example, the user's picture may be taken by the driver monitoring module 304. In yet another example, the driver's driving behavior may be used as an attribute. Furthermore, data related driver's driving behavior, such as the deviation of behavioral trends, spoken words, gaze monitoring and/or environmental conditions within the vehicle, etc. are used to provide useful data regarding the state of the driver.

Further in an embodiment of the invention, the driver monitoring module 304 helps in identifying driver profile and monitor driver's state. driver monitoring module 304 is a camera which can identify the driver whether it's an old person, a woman, a young boy, etc. Also, the module 304 has ability to identify various kinds of reactions of the driver. Whether the driver is happy, angry, sad, worried, tensed etc. The module 304 is also equipped with features to identify whether driver is attentive or not, is the driver sleepy, or looking at phone etc.

The environment condition module 306 acquires information from nearby surroundings of the vehicle. Various sensors, like RADAR, LiDAR, image sensors, ultrasonic sensors, infrared sensors, rain sensors, may be employed within the environment condition module 306. Information like traffic, lane markings, pavement, road signs, position of the vehicle with respect to surroundings, other objects around the vehicle, upcoming bad road conditions, vehicle to server communication, vehicle to vehicle communication etc. may be collected by the environment condition module 306.

The vehicle information module 308 acquires information regarding speed of the vehicle, or position of the vehicle, etc. Position of the vehicle may be sensed using a Global Positioning System (GPS) whereas speed may be ascertained by utilizing speed sensors affixed on the vehicle.

The processing module 310 gathers information from the drive mode module 302, the driver monitoring module 304, the environment condition module 306 and the vehicle information module 308 and processes the information for further usage. The processing module 310 processes information from the driver monitoring module 304 and determines whether to activate the warning module 312 or not. The activation is determined based on the analysis of the driver information received from the driver monitoring module 304. The processing module 310 determines plurality of physiological factors from the images captured and further analyzes the physiological factors.

In accordance with an embodiment of the invention, the processing module 310 analyzes the captured images and/or videos to determine the inattentiveness of the driver based on the first physiological factor from the plurality of physiological factors. Further, the second physiological factor is independently and/or simultaneously analyzed by the processing module 310 to support the first physiological factor. The plurality of physiological factors maybe heart rate readings, pupillary light reflex, skin conductance, pulse rate, respiratory rate, and breathing volume, etc. In an example, the heart rate of the driver is analyzed as the first physiological factor and pupillary light reflex, as the second physiological factor in a system to determine the alertness and inattentiveness of the driver. The physiological factors enable early prediction of drowsiness and inattentiveness of the drivers.

Further in an embodiment of the invention, the driver may pre-register his own profile with a server to provide base-line data to the processing module 310. The data may consist driver specific heart rate readings, skin conductance, pulse rate, respiratory rate and breathing volume, age, sex, eyesight, etc. The server of the system according to the present invention can store the created user profile and reuse it later in order to optimize the driving behavior of the vehicle. This data may also be used to compare and determine changes in driver physiological and behavioral factors.

In an embodiment of the invention, the driving behavior is determined from data received from the driver monitoring module 304, environment condition module 306, and vehicle information module 308. Also, the information of historical reactions may also be fetched from memory 314 for reaction time calculation. Determination of reaction time is done based on what state the driver is currently in, what are the physiological readings for the same and how the driver has been reacting to situations in the current journey. The reaction time may be utilized to better equip the system for any situations that may come up in the journey. The reaction time may also be compared to general reaction time of the driver as stored in the memory 314 to determine any anomalies and flag such situations to the system 300.

Further, the processing module 310 may initiate a warning to the driver for adjusting his reaction time based on the determined reaction time. The adjustment may be in the form of early reactions to certain situations or may be eased out reaction based on vehicle performance. For example if the driver has been pushing the brakes too hard then he may be provided an assistance to soften the brake controls or if the driver has been reacting late to curves, he may be provided a warning in next upcoming turns to act early in braking and steering control etc.

Further in an embodiment, the processing module 310 may identify certain sections of road, certain timings of the day, certain whether, certain environment, etc. wherein generally other drivers may have felt drowsy or sleepy or may have met some accident or may have affected the reaction times of the drivers. The processing module 310 may predict this from historical data stored that it may fetch from a remote server connected to it. Early predictive reaction time adjustments may be provided to the driver based on his current physiological and driving behavior.

The warning module 312, receives activation or deactivation instructions from the processing module 310. The warning module 312, on activation may display or present a warning to the driver through the display 316 that may be a screen of an infotainment system of the vehicle. The display 316 may be configured to receive inputs of the driver.

The inputs may be through a touch, physical button, a remote control, voice input, or gesture recognition. The display 316 may include a circuitry (not shown in figure) like a printed circuit board (PCB) or an integrated circuit containing appropriate components for receiving and recognizing the driver inputs. In accordance with another embodiment of the invention the warning may be provided through any other means like audio or visual.

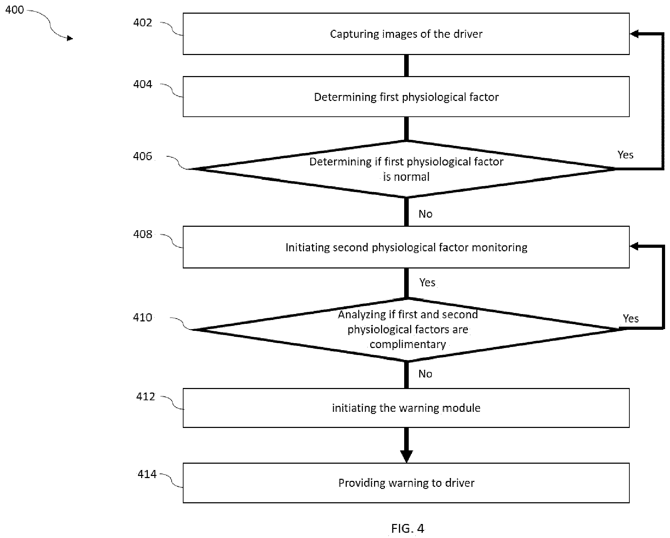

FIG. 4 is a flow chart of a method 400 for providing early warning to the driver. The order in which the method is described is not intended to be construed as a limitation, and any number of the described method blocks can be combined in any order to implement the method or alternate methods. Additionally, individual blocks may be deleted from the method without departing from the spirit and scope of the subject matter described herein. Furthermore, the method can be implemented in any suitable hardware, software, firmware, or combination thereof. However, for ease of explanation, in the embodiments described below, the method may be considered to be implemented in the above described system and/or the apparatus and/or any electronic device (not shown). The method starts at step 402 at which images of the driver are continuously captured using a camera. In another embodiment of the invention, short video segments may be taken at regular intervals of time. At step 404, the first physiological factor of the driver is determined using captured images and/or video of the driver at step 402. Further at step 406, the system determines whether the first physiological factor of the driver is within a threshold limit. If yes, then the method is returned to step 402 in order to keep monitoring the same. However, in case the first physiological factor is above the threshold limit then at step 408 the monitoring second physiological factor of the driver is initiated. Further, at step 410, it is determined, whether the first and second physiological factors are aligned within the threshold limit or not. If yes, then the method is returned to step 408 in order to re-monitor the second physiological factor of the driver. However, in case the first and second physiological factor of the driver are below the threshold then the method at step 412 initiates the warning module 312. Further to this, at step 410 the warning is provided to the driver.

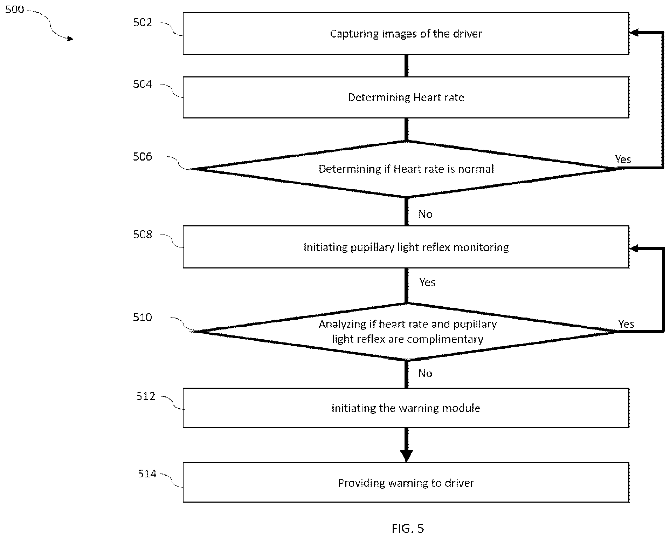

FIG. 5 is a flow chart of a method 500 for providing early warning to the driver, in accordance with an embodiment of the invention. The method 500 analyzes the heart rate and pupillary light reflex of the driver of the vehicle to monitor the driver's inattentiveness. The method starts at step 502 at which images of the driver are continuously captured using a camera. In another embodiment of the invention, short video segments may be taken at regular intervals of time. At step 504, the heart rate variability of the driver is determined using captured images and/or video of the driver at step 502. Further at step 506, the system determines whether the heart rate of the driver is within a threshold limit. If yes, then the method is returned to step 502 in order to keep monitoring the same. However, in case the heart rate is above the threshold limit then at step 508 the monitoring pupillary light reflex of the driver is initiated. Further, at step 510, it is determined, whether the heart rate and pupillary light reflex are aligned within the threshold limit or not. If yes, then the method is returned to step 508 in order to re-monitor the pupillary light reflex of the driver. However, in case the heart rate and pupillary light reflex of the driver are below the threshold then the method at step 512 initiates the warning module 312. Further to this, at step 514 a warning is also provided to the driver. The warning may be an audio warning, a visual warning, an audio-visual warning, haptic warning like vibration, etc.

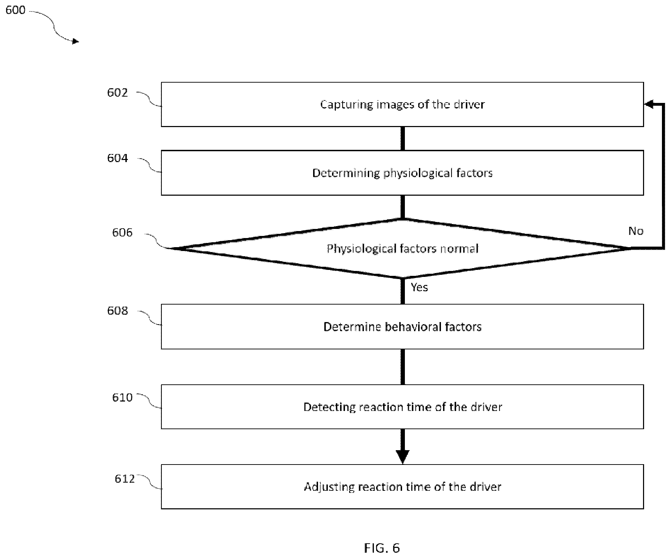

In an embodiment of the invention the FIG. 6 is a flow chart of a method 600 for detecting reaction time of driver during the drive. At step 602, images of the driver are captured using a camera. In another embodiment of the invention short video segments may be taken at regular intervals of time. At step 604, the processing module 310 analyzes the images of the driver to determine physiological factors i.e. heart rate readings of the driver. At step 606, it is determined whether the physiological factors are within normal range. If yes, then the method is returned to step 602 in order to keep monitoring the same. However, in case the physiological factors are not within the normal limits then the method at step 608 determines behavioral factors of the driver. Further to this, at step 610 reaction time of the driver is determined based on the physiological factors and the behavioral factors. At step 612, the reaction time of the driver is adjusted based on the determination. This may be done by providing a gentle warning to the driver.

It will be appreciated that, for clarity purposes, the above description has described embodiments of the present subject matter with reference to different functional units and processors. However, it will be apparent that any suitable distribution of functionality between different functional units, processors or domains may be used without detracting from the present subject matter.

The methods illustrated throughout the specification, may be implemented in a computer program product that may be executed on a computer. The computer program product may comprise a non-transitory computer-readable recording medium on which a control program is recorded, such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other tangible medium from which a computer can read and use.

Alternatively, the method may be implemented in transitory media, such as a transmittable carrier wave in which the control program is embodied as a data signal using transmission media, such as acoustic or light waves, such as those generated during radio wave and infrared data communications, and the like.

The terminology used herein is for the purpose of describing particular embodiments only and is not intended to be limiting of the disclosure. It will be appreciated that several of the above-disclosed and other features and functions, or alternatives thereof, may be combined into other systems or applications. Various presently unforeseen or unanticipated alternatives, modifications, variations, or improvements therein may subsequently be made by those skilled in the art without departing from the scope of the present disclosure as encompassed by the following claims.

## Claims

1. A method for monitoring a driver comprising:
capturing, a plurality of images of the driver, by a driver monitoring module (304);
storing the plurality of images of the driver in a memory (314);
determining a plurality of physiological factors of the driver to a plurality of situations experienced by the driver, based on the plurality of images of the driver by a processing module (310);
determining a plurality of behavioral factors of the driver to a plurality of situations experienced by the driver, based on the plurality of images of the driver by the processing module (310); and
detecting a reaction time of the driver by the processing module (310), to the plurality of situations experienced, based on the physiological factors and the behavioral factors.

2. The method of claim 1, **characterized in that** the plurality of physiological factors includes heart rate readings, pupillary light reflex, skin conductance, pulse rate, respiratory rate and breathing volume determined from captured images.

3. The method of claim 1, **characterized in that** the behavioral factors include braking time, steering turning time, deceleration initiation time determined from captured images.

4. A system (300) for monitoring a driver comprising;
at least one driver monitoring module (304) configured to capture a plurality of images of the driver;
a memory (314) connected to the driver monitoring module for storing the plurality of images of the driver;
a processing module (310) connected to the driver monitoring module configured to analyze the plurality of images of the driver for:
determining a plurality of physiological factors of the driver to a plurality of situations experienced by the driver, based on the images of the driver;
determining a plurality of behavioral factors of the driver to the plurality of situations experienced by the driver, based on the images of the driver; and
detecting a reaction time of the driver, to the plurality of situations experienced, based on the physiological factors and the behavioral factors.

5. The system (300) of claim 4, **characterized in that** the plurality of physiological factors includes heart rate readings, pupillary light reflex, skin conductance, pulse rate, respiratory rate and breathing volume determined from captured images.

6. The system (300) of claim 4, **characterized in that** the plurality of behavioral factors include braking time, steering turning time, deceleration initiation time determined from captured images.

7. The system (300) of claim 4, **characterized in that** the driver monitoring module (304) is a charge coupled device (CCD) camera.

8. The system (300) of claim 7, **characterized in that** the CCD camera monitors driver state based on eye gaze, blink rate of eyelids, change in skin tone, nostrils, jaw movements, frowning, baring teeth, movement of cheeks, movement of lips and head movements.

9. The system (300) of claim 4, **characterized in that** the processing module (310) is configured to identify relative changes in the plurality of physiological and behavioral factors and predict a warning based on the relative changes in the plurality of physiological factors and the behavioral factors.

10. The system (300) of claim 4, **characterized in that** the processing module (310) is connected to a remote server through a wireless communication protocol.
